# EUROPÄISCHE PATENTSCHRIFT

(11) **EP 2 404 916 B1**
(45) Veröffentlichungstag und Bekanntmachung des Hinweises auf die Patenterteilung: **17.06.2015**
(21) Anmeldenummer: 10168968.5
(22) Anmeldetag: 08.07.2010
(51) Int. Cl.: C07D 339/00, A61K 6/083

(54) **Dentalmaterialien auf der Basis von Dimersäure-Derivaten mit ringöffnend polymerisierbaren Gruppen**
Dental materials based on dimer acid derivatives with ring opening polymerisable groups
Matériaux dentaires à base de dérivés d'acide dimère dotés de groupes polymérisables à ouverture d'anneau

(43) Veröffentlichungstag der Anmeldung: 11.01.2012
(73) Patentinhaber: Ivoclar Vivadent AG, 9494 Schaan (LI)
(72) Erfinder: Moszner, Norbert, 9493, Mauren (LI); Angermann, Jörg, 7320, Sargans (CH); Fischer, Urs Karl, 9320, Arbon (CH); Rheinberger, Volker M., 9490, Vaduz (LI)
(74) Vertreter: UEXKÜLL & STOLBERG

(56) Entgegenhaltungen:
- WO-A1-2005/107626
- WO-A1-2008/124797
- JP-A- 8 300 523
- JP-A- 11 263 884
- US-A1- 2003 008 992
- US-A1- 2005 267 254
- US-A1- 2006 223 937
- JONSSON S ET AL: "Recent advances in photoinduced donor/acceptor copolymerization", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL LNKD- DOI:10.1016/S0168-583X(99)00110-X, Bd. 151, Nr. 1-4, 2. Mai 1999 (1999-05-02) , Seiten 268-278, XP004416440, ISSN: 0168-583X
- JÖNSSON ET AL.: "Mechanistic aspects of donor structure in maleimide/donor photo-coplymerizations", POLYMER PREPRINTS, Bd. 42, 2001, Seiten 703-704, XP8128566,

## Beschreibung

Die vorliegende Erfindung betrifft von hydrierten Dimersäuren abgeleitete Monomere mit ringöffnend polymerisierbaren Gruppen, die sich durch geringen Polymerisationsschrumpf auszeichnen und sich besonders für Dentalmaterialien eignen. Die Erfindung betrifft auch polymerisierbare Zusammensetzungen, die die erfindungsgemäßen Monomere enthalten, sowie deren Verwendung als Dentalmaterialien und zur Herstellung von Dentalwerkstoffen, insbesondere Kompositen, Zementen, Adhäsiven oder Beschichtungen.

Die Polymerisation von Monomeren wie Vinylverbindungen oder (Meth)acrylaten geht üblicherweise mit einer deutlichen Volumenkontraktion einher, die auch als Polymerisationsschrumpf bezeichnet wird. Dieser Polymerisationsschrumpf der verwendeten Monomere kann etwa bei Dentalmaterialien unter anderem zu nachteiligen Schrumpfungsspannungen und zur Randspaltbildung bei Füllungskompositen, zur verminderten Substrathaftung bei Befestigungskompositen oder Beschichtungsmaterialien sowie zur Beeinträchtigung der Dimensionsstabilität von Prothesenkunststoffen führen. Dementsprechend haben schrumpfungsarme Monomere im Dentalbereich großes Interesse gefunden (vgl. N. Moszner, U. Salz, Progress Polymer Sci. 26 (2001) 535-576 und N. Moszner, U. Salz, Macromol. Mater. Eng. 292 (2007) 245-271).

Als relativ schrumpfungsarme Monomere finden im Dentalbereich insbesondere die höhermolekularen Dimethacrylat-Vernetzer Bis-GMA und UDMA Anwendung, die einen Polymerisationsschrumpf ΔVp von 6,0 bzw. 6,1 Vol.-% zeigen. Allerdings weisen diese Vernetzer eine sehr hohe Viskosität auf (Bis-GMA: η = 800-1000 Pa·s; UDMA: η = 10 Pa·s), so dass sie meist in Mischung mit niedrigviskosen Dimethacrylatverdünnern wie Triethylenglycoldimethacrylat eingesetzt werden müssen, die aber wieder einen deutlich höheren Polymerisationsschrumpf aufweisen (Triethylenglycoldimethacrylat: ΔVp = 14,5 Vol.-%).

Radikalisch polymerisierbare cyclische Monomere zeichnen sich im Vergleich zu linearen Monomeren in der Regel durch einen geringeren Polymerisationsschrumpf aus (vgl. R. K. Sadhir, R. M. Luck, Expanding Monomers, CRC Press, Boca Raton etc. 1992). Allerdings weisen diese Monomere häufig eine gegenüber linearen Monomeren deutlich verringerte Reaktivität auf, wodurch ihre praktische Anwendbarkeit insbesondere im Dentalbereich deutlich eingeschränkt wird.

Als Dimersäuren werden allgemein cyclische, insbesondere mehrwertige Carbonsäuren bezeichnet, die durch Cyclodimerisierung ungesättigter Fettsäuren erhalten werden können. Typischerweise handelt es sich dabei um cyclische Di- oder Tricarbonsäuren, die etwa durch von Tonerde katalysierte Dimerisierung ungesättigter Fettsäuren, wie Ölsäure, Linolsäure oder Tallöl zugänglich sind. Solche Dimersäuren weisen üblicherweise durchschnittlich 36 C-Atome auf (vgl. Ullmann's Encyclopedia of Industrial Chemistry, 5th ed., Vol. A8, VCH, Weinheim and New York 1987, S. 535-536). Es sind Produkte mit hoher Reinheit kommerziell verfügbar, die beispielweise folgende ungesättigte Dimersäuren enthalten können:

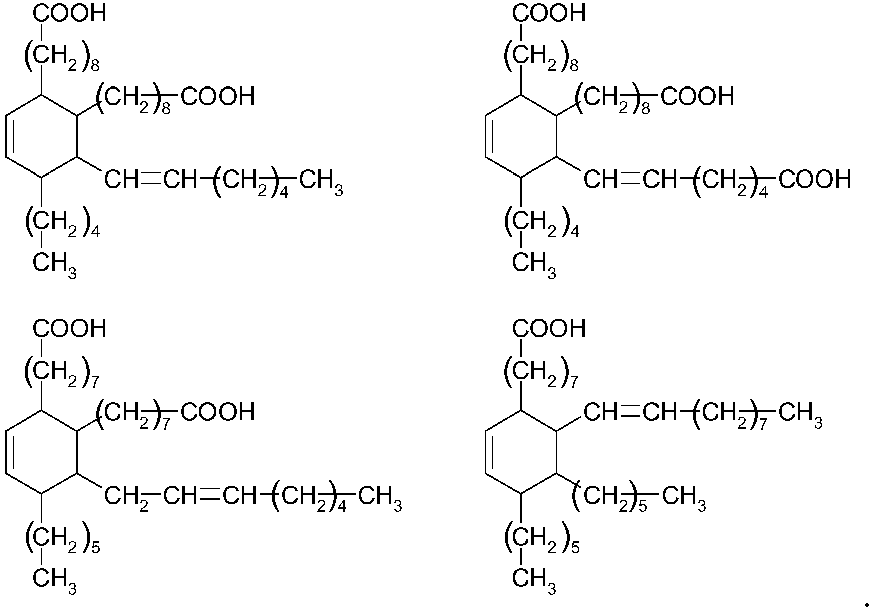

Durch Hydrierung ungesättigter Dimersäuren sind die entsprechenden hydrierten Dimersäuren zugänglich. Beispielsweise können durch Hydrierung der oben gezeigten ungesättigten Dimersäuren die folgenden hydrierten Dimersäuren erhalten werden:

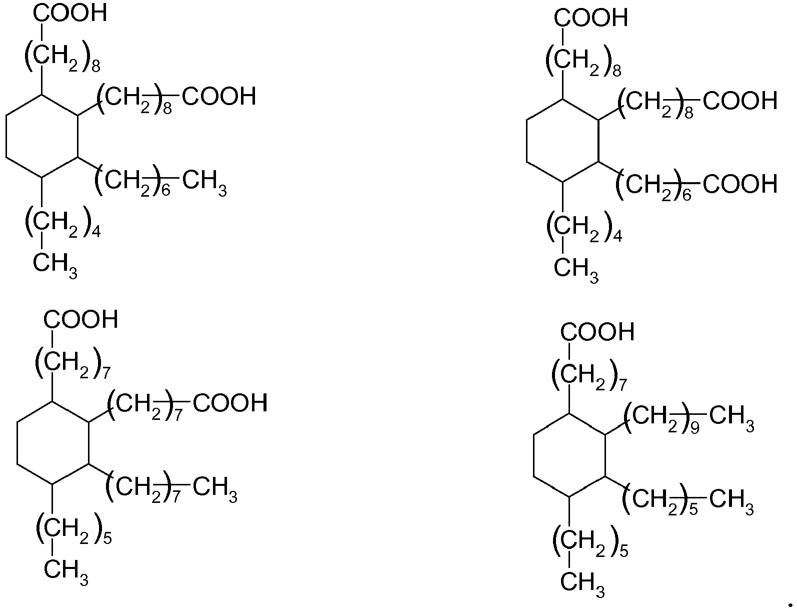

US 2008/0318188 A und WO 2005/107626 A1 beschreiben von Dimersäuren abgeleitete Dimethacrylate und deren Anwendung in Dentalzusammensetzungen. Diese zeigen eine geringe Schrumpfung bei der Polymerisation, ergeben jedoch Polymernetzwerke mit hoher Flexibilität und niedrigem E-Modul, was besonders für die Anwendung in Dentalmaterialien wie in Füllungskompositen nachteilig ist (vgl. M. Trujillo-Lemon, J. Ge, H. Lu, J. Tanaka, J.W. Stansbury, J. Polym. Sci., Part A: Polym. Chem. 44 (2006) 3921-3929).

Der Erfindung liegt daher die Aufgabe zugrunde, Monomere und auf diesen basierende Dentalmaterialien bereitzustellen, die einen deutlich geringeren Polymerisationsschrumpf zeigen und dabei im Vergleich zu den konventionell auf Methacrylaten basierenden Materialien vergleichbare mechanische Eigenschaften und Reaktivität insbesondere bei der radikalischen Photopolymerisation aufweisen.

Diese Aufgabe wird erfindungsgemäß durch ein radikalisch polymerisierbares Monomer der Formel (I) gelöst 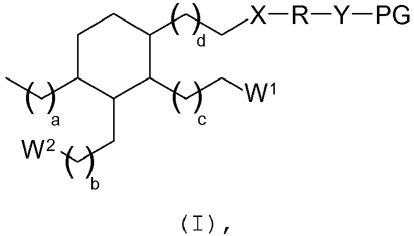 in der
- W¹ und W²: jeweils unabhängig für H oder X-R-Y-PG stehen, wobei mindestens eines von W¹ und W² für X-R-Y-PG steht,
- X: jeweils unabhängig entfällt oder für eine Ether-, Ester-, Amid-, Urethan- oder Harnstoffgruppe steht,
- Y: jeweils unabhängig entfällt oder für eine Ether-, Ester-, Amid-, Urethan- oder Harnstoffgruppe steht,
- R: jeweils unabhängig entfällt oder für einen C₁-C₁₆-Alkylenrest steht, der durch ein oder mehrere O-Atome unterbrochen sein kann, wobei R nur entfallen kann, wenn zugleich X und/oder Y entfällt,
- PG: jeweils unabhängig für eine cyclische, ringöffnend polymerisierbare Gruppe steht, und
- a, b, c und d: unabhängig voneinander die Werte 3 bis 10 annehmen können.

Durch die Formel (I) und die übrigen hierin gezeigten Formeln werden sämtliche konstitutions- und stereoisomeren Formen sowie Gemische verschiedener konstitutions- und stereoisomerer Formen, wie z.B. Racemate, erfasst. Die Formeln erfassen nur solche Verbindungen, die mit der chemischen Valenzlehre vereinbar sind.

Der Hinweis, dass ein Rest beispielsweise durch O-Atome unterbrochen sein kann, ist so zu verstehen, dass dieses Atome oder Gruppen in die Kohlenstoffkette des Restes eingeschoben werden, d.h. beidseitig von Kohlenstoffatomen begrenzt werden. Die Anzahl dieser Fremdatome oder Gruppen ist daher um mindestens 1 kleiner als die Zahl der Kohlenstoffatome, und die Fremdatome oder Gruppen können nicht endständig sein. Erfindungsgemäß sind in allen Fällen, in denen Reste Heteroatome enthalten können, Reste ohne Heteroatome bevorzugt.

Bevorzugte Monomere der Formel (I) sind solche, bei denen unabhängig voneinander
- W¹: für X-R-Y-PG steht,
- W²: für H steht,
- X: jeweils unabhängig entfällt oder für eine Ether-, Ester-, Amid- oder Urethangruppe steht,
- Y: jeweils unabhängig entfällt oder für eine Ether-, Ester-, Amid- oder Urethangruppe steht,
- R: jeweils unabhängig entfällt oder für einen C₁-C₁₂-Alkylenrest, insbesondere einen C₁-C₆-Alkylenrest und bevorzugt einen C₁-C₃-Alkylenrest steht, der durch ein oder mehrere O-Atome unterbrochen sein kann, wobei R nur entfallen kann, wenn zugleich X und/oder Y entfällt, und
- a, b, c und d: unabhängig voneinander die Werte 4 bis 9, insbesondere 4 bis 8, vorzugsweise 4 bis 7 und insbesondere die Werte 4, 5, 6 und 7 annehmen können.

Insbesondere sind solche Monomere der Formel (I) bevorzugt, bei denen
- W¹: für X-R-Y-PG steht,
- W²: für H steht,
- X: jeweils unabhängig entfällt oder für eine Ether-, Ester-, Amid-, oder Urethangruppe steht,
- Y: jeweils unabhängig entfällt oder für eine Ether-, Ester-, Amid-, oder Urethangruppe steht,
- R: jeweils unabhängig entfällt oder für einen C₁-C₁₂-Alkylenrest steht, der durch ein oder mehrere O-Atome unterbrochen sein kann, wobei R nur entfallen kann, wenn zugleich X und/oder Y entfällt, und
- a, b, c und d: unabhängig voneinander die Werte 4, 5, 6 und 7 annehmen können.

In einer Ausführungsform handelt es sich bei den Monomeren der Formel (I) um solche der Formel (Ia) 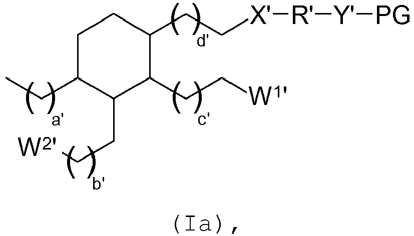 in der
- W¹' und W²': jeweils unabhängig für CH₃ oder X'-R'-Y'-PG stehen, wobei mindestens eines von W¹' und W²' für X'-R'-Y'-PG steht,
- X': jeweils unabhängig entfällt oder für -CH₂-O-, -C(O)-O-, -CH₂-O-C(O)-, -C(O)-NH-, -CH₂-NH-C(O)-, -CH₂-NH-C(O)-O-, -CH₂-O-C(O)-NH- oder -CH₂-NH-C(O)-NH- steht,
- Y': jeweils unabhängig entfällt oder für -O-, -C(O)-O-, -O-C(O)-, -C(O)-NH-, -NH-C(O)-, -NH-C(O)-O-, -O-C(O)-NH- oder -NH-C(O)-NH- steht,
- R': jeweils unabhängig entfällt oder für einen C₁-C₁₆-Alkylenrest, insbesondere einen C₁-C₁₂-Alkylenrest, bevorzugt einen C₁-C₆-Alkylenrest und am meisten bevorzugt einen C₁-C₃-Alkylenrest steht, der durch ein oder mehrere O-Atome unterbrochen sein kann, wobei R' nur entfallen kann, wenn zugleich X' und/oder Y' entfällt,
- PG: jeweils unabhängig für eine cyclische, ringöffnend polymerisierbare Gruppe steht, und
- a', b', c' und d': unabhängig voneinander die Werte 3 bis 10, insbesondere 4 bis 9, vorzugsweise 4 bis 8, weiter bevorzugt 4 bis 7 und insbesondere die Werte 4, 5, 6 und 7 annehmen können.

Es ist bevorzugt, dass W¹' für X'-R'-Y'-PG und W²' für CH₃ steht.

Bevorzugte Monomere der Formel (Ia) sind solche, bei denen mindestens eine und vorzugsweise alle der folgenden Variablen die angegebenen Werte haben:
- a': ist 3 bis 10 und vorzugsweise 4 oder 5,
- b': ist 3 bis 10, insbesondere 3 bis 9, vorzugsweise 3 bis 6 und am meisten bevorzugt 4, 5 oder 6,
- c': ist 3 bis 10, insbesondere 3 bis 9, vorzugsweise 6 bis 9 und am meisten bevorzugt 6, 7 oder 8,
- d': ist 3 bis 10 und vorzugsweise 6 oder 7.

Es ist insbesondere bevorzugt, dass die Summe von a' und d' 11 beträgt und/oder dass die Summe von b' und c' 12 beträgt.

Erfindungsgemäße ringöffnend polymerisierbare Gruppen PG sind: 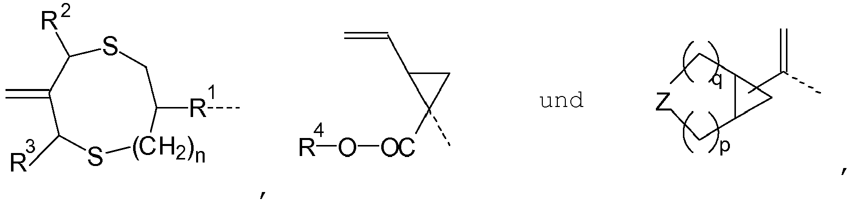 in denen
- Z: ausgewählt ist aus O, S, 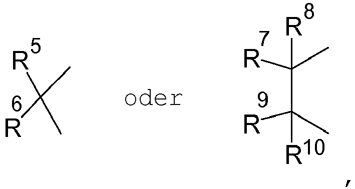
- R¹: entfällt oder für einen C₁-C₁₆-Alkylenrest steht, der durch ein oder mehrere O-Atome unterbrochen sein kann,
- R²: für H oder einen C₁-C₁₀-Alkylrest steht,
- R³: für H oder einen C₁-C₁₀-Alkylrest steht,
- R⁴: für H oder einen C₁-C₁₀-Alkylrest steht,
- R⁵: für H, -CO-OR¹¹, -CO-R¹¹ oder R¹¹ steht,
- R⁶: für H, -CO-OR¹¹, -CO-R¹¹ oder R¹¹ steht,
- R⁷ bis R¹⁰: jeweils unabhängig für H, -CO-OR¹¹, -CO-R¹¹, einen C₁-C₁₅-Alkylrest, der durch ein oder mehrere O-Atome unterbrochen sein kann, einen cycloaliphatischen C₄-C₁₂-Rest, einen bicyclischen C₅-C₁₂-Rest, einen C₆-C₁₄-Arylrest oder einen C₇-C₂₀-Arylalkyl-Rest stehen,
- R¹¹: für einen C₁-C₁₅-Alkylrest, der durch ein oder mehrere O-Atome unterbrochen sein kann, einen cycloaliphatischen C₄-C₁₂-Rest, einen bicyclischen C₅-C₁₂-Rest, einen C₆-C₁₄-Arylrest oder einen C₇-C₂₀-Arylalkyl-Rest steht,
- n: 0 oder 1 ist,
- p: 0, 1, 2 oder 3 ist und
- q: 0, 1, 2 oder 3 ist.

Ganz besonders bevorzugte ringöffnend polymerisierbare Gruppen PG sind: 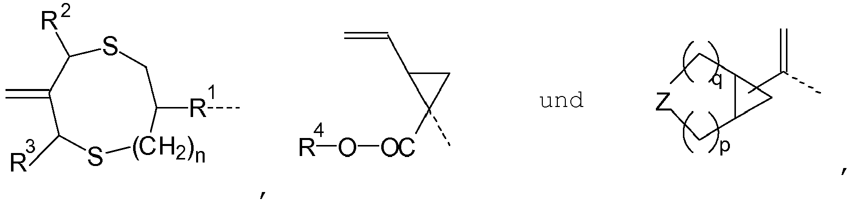 in denen
- Z: ausgewählt ist aus O, S, 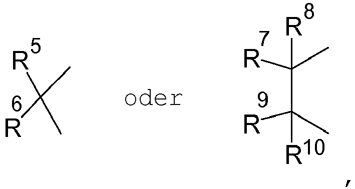
- R¹: entfällt oder für einen C₁-C₁₀-Alkylenrest steht, der durch ein oder mehrere O-Atome unterbrochen sein kann,
- R²: für H oder einen C₁-C₅-Alkylrest steht,
- R³: für H oder einen C₁-C₅-Alkylrest steht,
- R⁴: für H oder einen C₁-C₅-Alkylrest steht,
- R⁵: für H, -CO-OR¹¹, -CO-R¹¹ oder R¹¹ steht,
- R⁶: für H, -CO-OR¹¹, -CO-R¹¹ oder R¹¹ steht,
- R⁷ bis R¹⁰: jeweils unabhängig für H, -CO-OR¹¹, -CO-R¹¹, einen C₁-C₁₅-Alkylrest, der durch ein oder mehrere O-Atome unterbrochen sein kann, einen cycloaliphatischen C₄-C₆-Rest, einen bicyclischen C₅-C₁₂-Rest, einen Phenyl-Rest oder einen Benzyl-Rest stehen,
- R¹¹: für einen C₁-C₁₀-Alkylrest, der durch ein oder mehrere O-Atome unterbrochen sein kann, einen cycloaliphatischen C₄-C₆-Rest, einen Phenyl-Rest oder einen Benzyl-Rest steht,
- n: 0 oder 1 ist,
- p: 0, 1 oder 2 ist und
- q: 0, 1 oder 2 ist.

Es hat sich gezeigt, dass sich die erfindungsgemäßen Monomere durch einen außerordentlich geringen Polymerisationsschrumpf auszeichnen und daher besonders zur Herstellung schrumpfungsarmer Dentalmaterialien geeignet sind. Dabei hat sich überraschend gezeigt, dass Dentalmaterialien auf Basis der erfindungsgemäßen Monomere hervorragende mechanische Eigenschaften und hohe Reaktivität insbesondere bei der radikalischen Photopolymerisation aufweisen, die mit konventionellen Dentalmaterialien auf Basis von Methacrylaten vergleichbar sind.

Die erfindungsgemäßen Monomere der Formel (I) können ausgehend von geeignet funktionalisierten cyclischen Monomeren durch Umsetzung mit einer hydrierten Dimersäure oder einem geeignet funktionalisierten Derivat davon erhalten werden.

So können Monomere, bei denen X für eine Estergruppe steht, durch Umsetzung einer hydrierten Dimersäure mit einem cyclischen Monomeren der Formel HO-R-Y-PG erhalten werden:

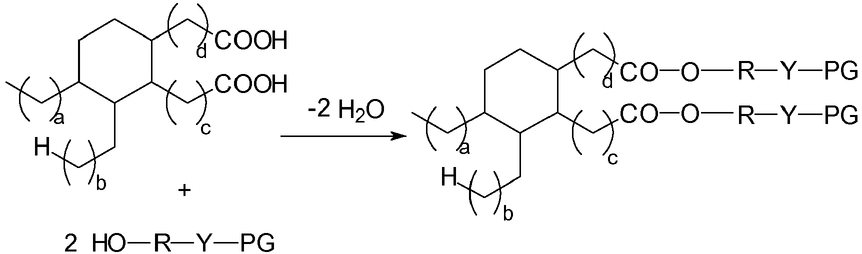

### Konkretes Beispiel:

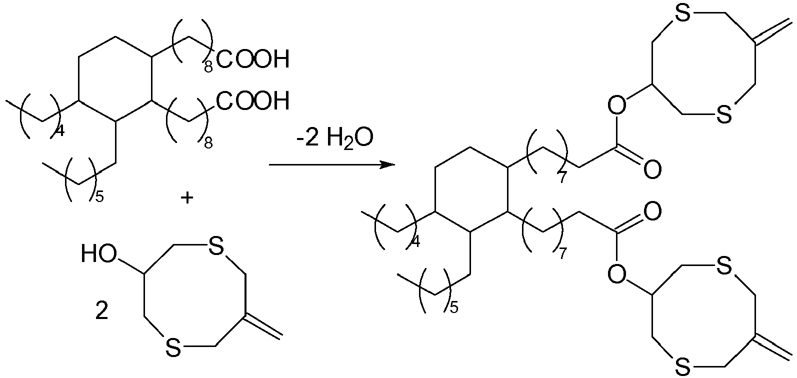

In anderen Fällen ist es gegebenenfalls notwendig, eine oder mehrere Carbonsäuregruppen der hydrierten Dimersäure in eine geeignete andere funktionelle Gruppen zu überführen. Entsprechende Methoden sind aus der organischen Chemie grundsätzlich bekannt. Geeignete Methoden zur Überführung der Carbonsäuregruppen von hydrierten Dimersäuren in andere funktionelle Gruppen werden beispielsweise auch in US 2008/0318188 A angesprochen.

Geeignet funktionalisierte cyclische Monomere für die Synthese der erfindungsgemäßen Monomere der Formel (I) sind aus der Literatur bekannt. Beispielsweise ist die Synthese von Vinylcyclopropanen bzw. von bicyclischen Cyclopropylacrylaten beschrieben von N. Moszner et al. in Macromol. Rapid. Commun. 18 (1997) 775-780 bzw. von A. de Meijere et al. in Eur. J. Org. Chem. (2004) 3669-3678, während die Synthese von funktionalisierten cyclischen Allylsulfiden z.B. von R. A. Evans und E. Rizzardo in J. Polym. Sci., Part A. Polym. Chem. 39 (2001) 202-215 und Macromolecules 33 (2000) 6722-6731 beschrieben wurde.

Einige Beispiele der erfindungsgemäßen Monomere der Formel (I) sind nachfolgend aufgeführt:

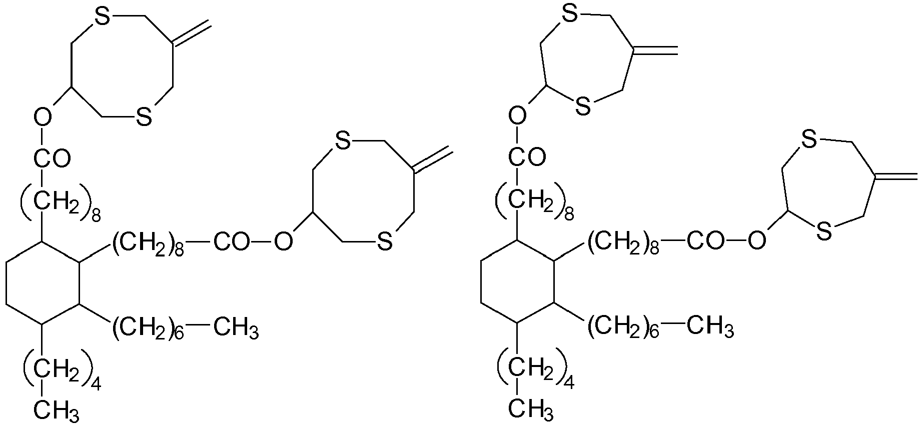

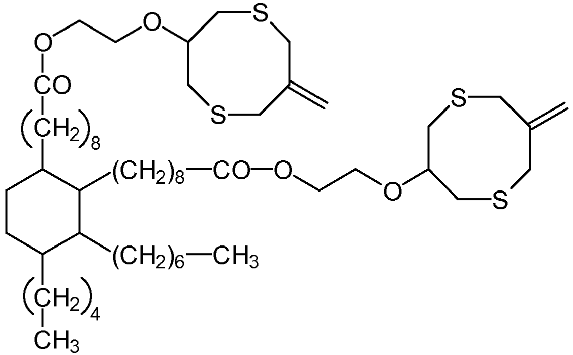

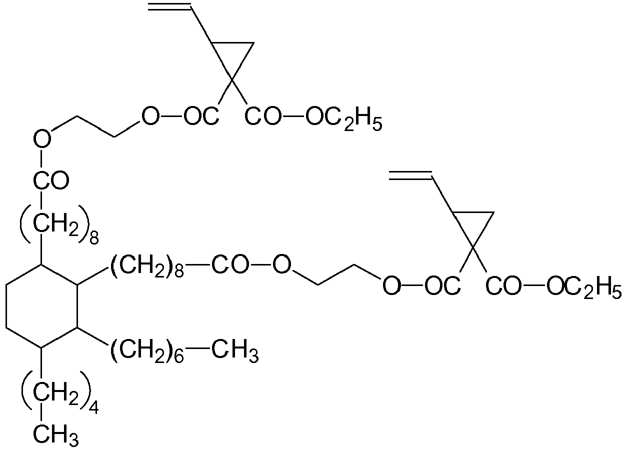

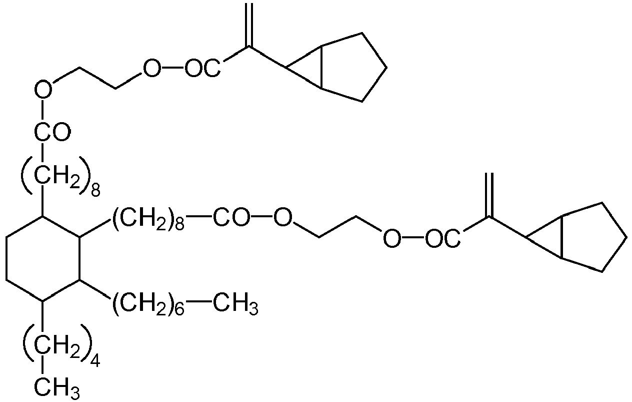

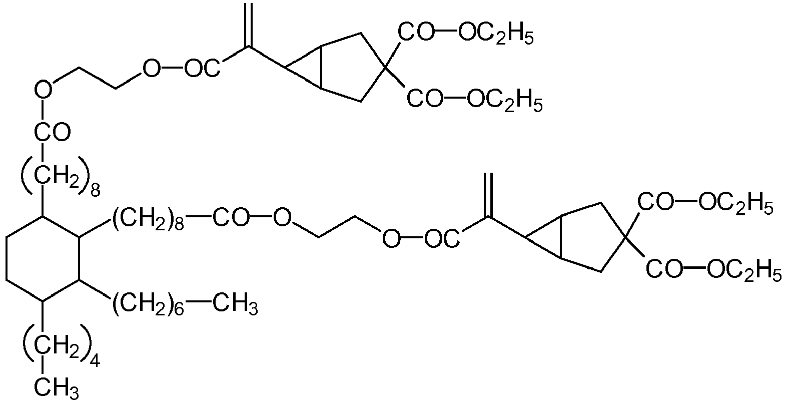

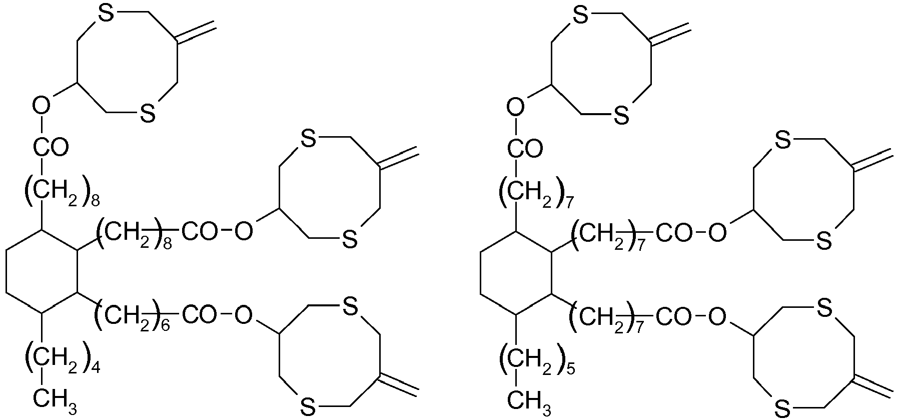

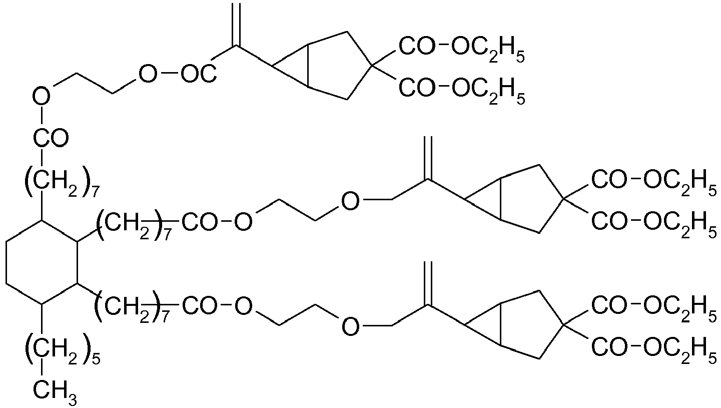

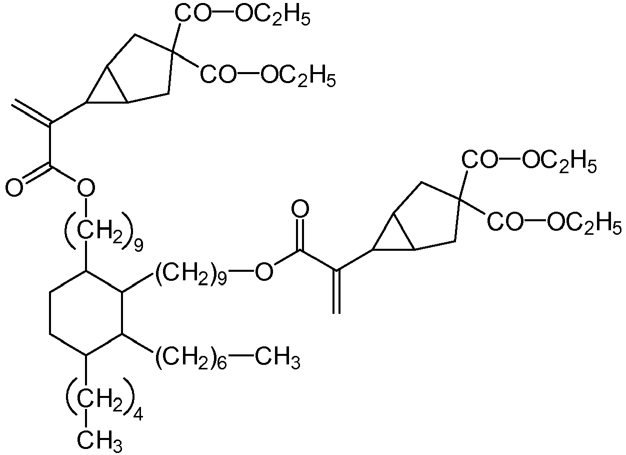

Die Erfindung betrifft auch eine polymerisierbare Zusammensetzung, die mindestens ein Monomer der obigen Formel (I) enthält.

Die erfindungsgemäßen Zusammensetzungen auf Basis der Monomere der Formel (I) lassen sich mit den bekannten radikalischen Initiatoren polymerisieren, wie beispielsweise den in Encyclopedia of Polymer Science and Engineering, Vol. 13, Wiley-Intersci. Pub., New York etc. 1988, 754ff. beschriebenen. Es eignen sich besonders Photoinitiatoren, wie die aus J.P. Fouassier, J.F. Rabek (Hrsg.), Radiation Curing in Polymer Science and Technology, Vol. II, Elsevier Applied Science, London und New York 1993, bekannten. Für den UV- oder sichtbaren Bereich sind Benzoinether, Dialkylbenzilketale, Dialkoxyacetophenone, Acyl- oder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon als Photoinitiatoren bevorzugt. Besonders geeignet sind auch Norrish-Typ-I-Photoinitiatoren, vor allem Acyl- und Bisacylphosphinoxide sowie Monoacyltrialkyl- und Diacyldialkylgermanium-Verbindungen, wie Benzoyltrimethylgermanium, Dibenzoyldiethylgermanium oder Bis-(4-methoxybenzoyl)-diethylgermanium. Geeignete Germanium-Initiatoren sind beispielsweise in EP 1 905 413 und EP 2 103 297 beschrieben. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Weiterhin können auch Azoverbindungen, wie 2,2'-Azobis-(isobutyronitril) (AIBN) oder Azobis-(4-cyanovaleriansäure), oder Peroxide, wie Dibenzoylperoxid, Dilauroylperoxid, tert.-Butylperoctoat, tert.-Butylperbenzoat oder Di-(tert.-butyl)-peroxid, als Initiatoren für die radikalische Polymerisation verwendet werden. Als Initiatoren für die Heißhärtung eignen sich besonders Benzpinakol und 2,2'-Dialkylbenzpinakole.

Zur Beschleunigung der Initiierung werden Peroxide und α-Diketone vorzugsweise in Kombinationen mit aromatischen Aminen eingesetzt. Bevorzugte Redoxsysteme sind Kombinationen aus Benzoylperoxid oder Campherchinon mit Aminen, wie N,N-Dimethyl-p-toluidin, N,N-Dihydroxyethyl-p-toluidin, p-Dimethylaminobenzoesäureethylester oder strukturverwandten Systemen. Darüber hinaus sind auch Redoxsysteme geeignet, die Peroxide in Kombination mit Ascorbinsäure, Barbituraten oder Sulfinsäuren enthalten.

Die erfindungsgemäßen Zusammensetzungen können ein oder mehrere Monomere der Formel (I) enthalten. Sie können darüber hinaus neben den Monomeren der Formel (I) weitere radikalisch polymerisierbare Monomere mit einer oder mehreren radikalisch polymerisierbaren Gruppen enthalten. Dentalwerkstoffe, die mindestens ein weiteres radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Bevorzugte zusätzliche Monomere sind mono- oder mehrfach funktionelle (Meth)acrylate oder (Meth)acrylamide ((Meth)acrylverbindungen). Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden.

Bevorzugte mehrfach funktionelle Monomere sind Bisphenol-A-di(meth)acrylat, Bis-GMA (ein Additionsprodukt aus Methacrylsäure und Bisphenol-A-diglycidylether), ethoxyliertes Bisphenol-A-di (meth) acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat sowie Butandioldi(meth)acrylat, 1,10-Decandioldi(meth)acrylat oder 1,12-Dodecandioldi(meth)acrylat.

Ganz besonders bevorzugt sind Mischungen von Monomeren der Formel (I) in Mischung mit bekannten schrumpfungsarmen radikalisch ringöffnend polymerisierbaren Monomeren wie mono- oder multifunktionellen Vinylcyclopropanen bzw. bicyclischen Cyclopropanderivaten, vorzugsweise den in DE 196 16 183 C2 und EP 1 413 569 A1 offenbarten Monomeren, oder cyclischen Allylsulfiden, vorzugsweise den in US 6,043,361 und US 6,344,556 offenbarten Monomeren. Weiter bevorzugt sind außerdem Mischungen von Monomeren der Formel (I) mit mindestens einem weiteren ringöffnend polymerisierbaren Monomer und mindestens einem radikalisch polymerisierbaren Monomer mit zwei oder mehr radikalisch polymerisierbaren Gruppen, insbesondere den oben aufgeführten mehrfach funktionellen (Meth)acrylatverbindungen.

Besonders bevorzugte ringöffnend polymerisierbare Monomere sind Vinylcyclopropane, wie 1,1-Di(ethoxycarbonyl)- oder 1,1-Di(methoxy-carbonyl)-2-vinylcyclopropan oder die Ester der 1-Ethoxycarbonyl-oder 1-Methoxycarbonyl-2-vinylcyclopropan-carbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0] hex-1-yl)acrylsäuremethyl- oder -ethylester oder deren Disubstitutionsprodukte in 3-Stellung wie (3,3-Bis-(ethoxycarbonyl)bicyclo [3.1.0]hex-1-yl)acrylsäuremethyl- oder -ethylester. Bevorzugte cyclische Allylsulfide sind insbesondere die Additionsprodukte von 2-(Hydroxymethyl)-6-methylen-1,4-dithiepan oder 7-Hydroxy-3-methylen-1,5-dithiacylooctan mit 2,2,4-Trimethylhexymethylen-1,6-disisocyanat oder einem asymmetrischen Hexamethylendiisocyanat-Trimeren wie Desmodur XP 2410 der Bayer AG.

Weiterhin können die erfindungsgemäßen Zusammensetzungen auf der Basis der Monomere der Formel (I) einen oder mehrere Füllstoffe enthalten, vorzugsweise organische oder anorganische partikuläre Füllstoffe. Füllstoffe mit einem mittleren Partikeldurchmesser von 10 nm bis 5 µm sind besonders geeignet. Bevorzugte anorganische partikuläre Füllstoffe sind amorphe kugelförmige nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂ vorzugsweise mit einem mittleren Partikeldurchmesser von 10 bis 200 nm, mikrofeine Füllstoffe, Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Glasfasern, Polyamid- oder Kohlenstoff-Fasern eingesetzt werden.

Schließlich lassen sich den erfindungsgemäßen Zusammensetzungen auf der Basis der Monomere der Formel (I) im Bedarfsfall weitere Additive zusetzen, wie z.B. Stabilisatoren, UV-Absorber, Farbstoffe oder Pigmente sowie Lösungsmittel oder Gleitmittel.

Erfindungsgemäß sind solche Zusammensetzungen bevorzugt, die die folgenden Komponenten enthalten:
(a) 1 bis 90 Gew.-%, bevorzugt 1 bis 80 Gew.-% und besonders bevorzugt 5 bis 70 Gew.-% Monomer der Formel (I),
(b) 0,01 bis 10 Gew.-%, besonders bevorzugt 0,1 bis 3,0 Gew.-% Initiator für die radikalische Polymerisation,
(c) 0 bis 80 Gew.-%, bevorzugt 0 bis 60 Gew.-% und besonders bevorzugt 5 bis 50 Gew.-% weitere radikalisch polymerisierbare Monomere, vorzugsweise 5 bis 80 Gew.-% weiteres ringöffnend polymerisierbares Monomer und 0 bis 50 Gew.-% mehrfach funktionelles (Meth)acrylat,
(d) 0 bis 85 Gew.-% Füllstoff,
(e) ggf. 0,01 bis 50 Gew.-%, bevorzugt 0,05 bis 5 Gew.-% und besonders bevorzugt 0,1 bis 2 Gew.-% Additiv und
(f) 0 bis 95 Gew.-%, bevorzugt 0 bis 70 Gew.-% und besonders bevorzugt 0 bis 50 Gew.-% Lösungsmittel.

Alle Prozentangaben beziehen sich, wenn nicht anders angegeben, auf die Gesamtmasse der Zusammensetzung.

Die erfindungsgemäßen Zusammensetzungen eignen sich besonders als Dentalmaterialien, insbesondere als Komposit, Zement, Adhäsiv oder Beschichtungsmaterial.

Die bevorzugte Zusammensetzung der Materialien für dentale Anwendungen richtet sich nach dem gewünschten Verwendungszweck.

Bevorzugte Adhäsive und Beschichtungsmaterialien enthalten die folgenden Komponenten:
(a) 1 bis 90 Gew.-%, bevorzugt 1 bis 80 Gew.-% und besonders bevorzugt 5 bis 70 Gew.-% Monomer der Formel (I),
(b) 0,01 bis 10 Gew.-%, besonders bevorzugt 0,1 bis 2,0 Gew.-% Initiator für die radikalische Polymerisation,
(c) 0 bis 80 Gew.-%, bevorzugt 0 bis 60 Gew.-% und besonders bevorzugt 5 bis 50 Gew.-% weitere radikalisch polymerisierbare Monomere, vorzugsweise 5 bis 80 Gew.-% weiteres ringöffnend polymerisierbares Monomer und 0 bis 50 Gew.-% mehrfach funktionelles (Meth)acrylat,
(d) 0 bis 85 Gew.-% und bevorzugt 0 bis 20 Gew.-% Füllstoff,
(e) ggf. 0,01 bis 50 Gew.-%, bevorzugt 0,05 bis 5 Gew.-% und besonders bevorzugt 0,1 bis 2 Gew.-% Additiv und
(f) 0 bis 95 Gew.-%, bevorzugt 0 bis 70 Gew.-% und besonders bevorzugt 0 bis 50 Gew.-% Lösungsmittel.

Bevorzugte Zemente enthalten die folgenden Komponenten:
(a) 1 bis 90 Gew.-%, bevorzugt 1 bis 70 Gew.-% und besonders bevorzugt 5 bis 30 Gew.-% Monomer der Formel (I),
(b) 0,01 bis 10 Gew.-%, besonders bevorzugt 0,1 bis 2,0 Gew.-% Initiator für die radikalische Polymerisation,
(c) 0 bis 80 Gew.-%, bevorzugt 0 bis 50 Gew.-% und besonders bevorzugt 5 bis 30 Gew.-% weitere radikalisch polymerisierbare Monomere, vorzugsweise 5 bis 30 Gew.-% weiteres ringöffnend polymerisierbares Monomer und 0 bis 10 Gew.-% mehrfach funktionelles (Meth)acrylat,
(d) 0 bis 85 Gew.-% und besonders bevorzugt 20 bis 70 Gew.-% Füllstoff und
(e) ggf. 0,01 bis 50 Gew.-%, bevorzugt 0,05 bis 5 Gew.-% und besonders bevorzugt 0,1 bis 5 Gew.-% Additiv.

Bevorzugte Komposite enthalten die folgenden Komponenten:
(a) 1 bis 90 Gew.-%, bevorzugt 1 bis 70 Gew.-% und besonders bevorzugt 5 bis 20 Gew.-% Monomer der Formel (I),
(b) 0,01 bis 10 Gew.-%, besonders bevorzugt 0,1 bis 2,0 Gew.-% Initiator für die radikalische Polymerisation,
(c) 0 bis 80 Gew.-%, bevorzugt 0 bis 50 Gew.-% und besonders bevorzugt 5 bis 20 Gew.-% weitere radikalisch polymerisierbare Monomere, vorzugsweise 5 bis 20 Gew.-% weiteres ringöffnend polymerisierbares Monomer und 0 bis 10 Gew.-% mehrfach funktionelles (Meth)acrylat,
(d) 0 bis 85 Gew.-% und besonders bevorzugt 20 bis 85 Gew.-% Füllstoff und
(e) ggf. 0,01 bis 50 Gew.-%, bevorzugt 0,05 bis 5 Gew.-% und besonders bevorzugt 0,1 bis 2 Gew.-% Additiv.

Die vorliegende Erfindung betrifft auch die Verwendung von radikalisch polymerisierbaren Monomeren der Formel (I) bzw. polymerisierbaren Zusammensetzungen, die mindestens ein Monomer der Formel (I) enthalten, als Dentalmaterial und insbesondere zur Herstellung von Dentalwerkstoffen, vorzugsweise den oben beschriebenen Dentalwerkstoffen.

Außerdem betrifft die Erfindung ein Verfahren zur Herstellung von Formkörpern, wie Kronen, Brücken, Inlays und künstlichen Zähnen, bei dem man eine erfindungsgemäße Zusammensetzung auf an sich bekannte Weise zu dem Formkörper formt und dann zumindest teilweise, vorzugsweise vollständig, härtet. Die Härtung erfolgt vorzugsweise durch radikalische Polymerisation.

Die Erfindung wird im Folgenden anhand von Beispielen näher erläutert.

### Beispiele:

### Beispiel 1:

### Synthese eines ringöffnend polymerisierbaren hydrierten Dimersäure-Derivates RODA

Zu einer Lösung von 17,63 g (0,100 mol) 7-Methylen-1,5-dithiacyclooctan-3-ol, 28,25 g (0,050 mol) Dimersäure und 0,611 g (0,005 mol) 4-Dimethylaminopyridin in 200 ml wasserfreiem Methylenchlorid wurden bei 0 °C portionsweise 21,09 g (0,110 mol) N-(3-Dimethylaminopropyl)-N'-ethyl-carbodiimid-hydrochlorid innerhalb von 2 h zugegeben. Nach 6 h Rühren bei 0 °C wurde das Reaktionsgemisch mit 500 ml Wasser versetzt. Nach der Phasentrennung wurde die organische Phase mit 2 N Salzsäure (2 x 100 ml) und Wasser (2 x 100 ml) gewaschen, mit wasserfreiem Natriumsulfat getrocknet und vom Lösungsmittel befreit. Nach einer chromatographischen Reinigung über Kieselgel (Hexan/Ethylacetat 4:1) wurden 24,10 g (55 %) einer farblosen, viskosen Flüssigkeit (η = 7,70 Pa·s) erhalten.
¹H-NMR (400 MHz, CDCl₃) : δ (ppm) = 0, 83-0, 90, 1, 04-1, 47, 1, 57-1,60 (3 m, 62H, CH, CH₂, CH₃; *a*), 2,27 (t, J = 7,5 Hz, 4H, O=C-CH₂; *b*), 3,02 und 3,03 (2 d, J = 1,68 Hz, 3,28 Hz, je 8H, SCH₂-CHO; *c*), 3,24 (d, J = 2,6 Hz, 8H, SCH₂-C=; *d*), 4,99-5,05 (m, 2H, OCH; e), 5,24 (s, 4H, =CH₂; *f*). 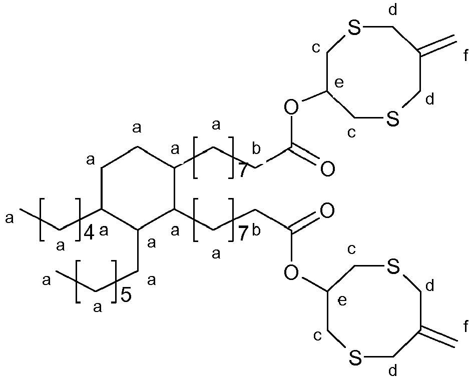 ¹³C-NMR (100 MHz, CDCl₃) : δ (ppm) = 14,1 (CH₃; a), 22,7-34,4 (CH, CH₂; *b*), 32,7, 37,9 (SCH₂; *c*), 72,3 (OCH; *d*), 120,4 (=CH₂; *e*), 145,0 (C=CH₂; *f*), 173,0 (C=O; *g*). 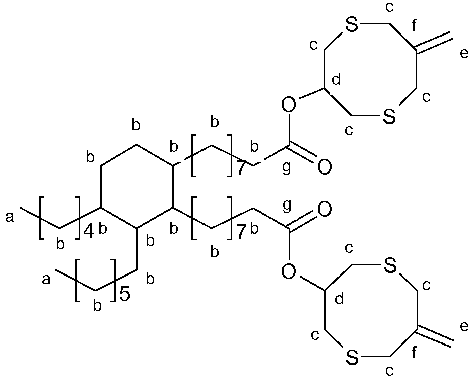 IR (Diamant-ATR) : ν = 3060 (w, =CH), 2921, 2852 (s, CH₂, CH₃), 1734 (s, C=O), 1633 (w, C=C), 1457 (m, CH₂, CH₃), 1377 (m, CH₃), 1160, 1092 (s, CSC, COC), 903 cm⁻¹ (s, =CH).

### Beispiel 2:

### Synthese eines methacrylierten Dimersäure-Derivates DMDA (Vergleichsbeispiel)

Zu einer Lösung von 45,20 g (0,080 mol) Dimersäure und 0,438 g (0,006 mol) N,N-Dimethylformamid in 250 ml wasserfreiem Toluol wurden bei Raumtemperatur 30,46 g (0,240 mol) Oxalylchlorid zugetropft. Nach 2 h wurden das überschüssige Oxalylchlorid und das Lösungsmittel abdestilliert (60 °C, 40 mbar). Der erhaltene braune Rückstand wurde in 250 ml wasserfreiem Methylenchlorid aufgenommen und bei Raumtemperatur zu einer Lösung von 20,82 g (0,160 mol) 2-Hydroxyethyl-methacrylat und 17,0 g (0,168 mol) Triethylamin in 250 ml wasserfreiem Methylenchlorid getropft. Nach 1 h wurde das Reaktionsgemisch mit 1 N Salzsäure (2 x 100 ml), gesättigter NatriumhydrogencarbonatLösung (2 x 100 ml) und Wasser (2 x 100 ml) gewaschen, die organische Phase mit wasserfreiem Natriumsulfat getrocknet und das Lösungsmittel im Vakuum entfernt. Nach einer chromatographischen Reinigung über Kieselgel (Methylenchlorid/Ethylacetat 4:1) wurden 31,20 g (51 %) einer orangefarbenen, leicht viskosen Flüssigkeit (η = 0,35 Pa·s) erhalten.
¹H-NMR (400 MHz, CDCl₃): δ (ppm) = 0,83-0,90, 1,04-1,47, 1,60-1,64 (3 m, 62H, CH, CH₂, CH₃; *a*), 1,95 (s, 6H, CH₃; *b*), 2,32 (t, J = 7,5 Hz, 4H, 0=C-CH₂; *c*), 4,31-4,36 (m, 8H, OCH₂; *d*), 5, 59, 6, 13 (2 s, je 2H, =CH₂; e). 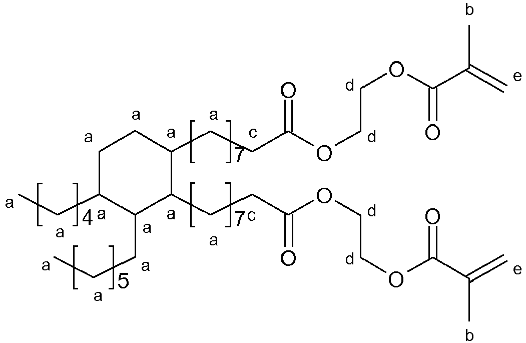 ¹³C-NMR (100 MHz, CDCl₃): δ (ppm) = 14,1, 18,3 (CH₃; a), 22,7-34,2 (CH, CH₂; *b*), 61,9, 62,5 (OCH₂; *c*), 126,0 (=CH₂; *d*), 136,0 (C=CH₂; e), 167, 1, 173, 0 (C=O; *f*). 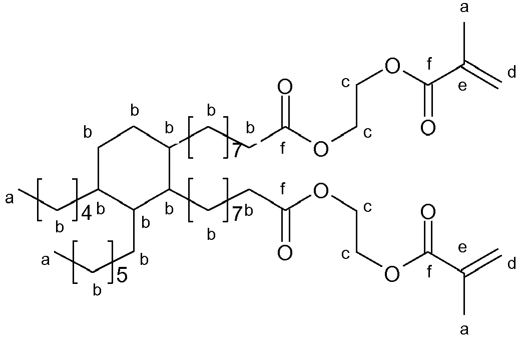 IR (Diamant-ATR): ν = 3090 (w, =CH), 2923, 2853 (s, CH₂, CH₃), 1735, 1722 (s, C=O), 1638 (w, C=C), 1455 (m, CH₂, CH₃), 1376 (m, CH₃), 1152 (vs, COC), 939 cm⁻¹ (m, =CH).

### Beispiel 3:

### Herstellung eines Dentalkomposits auf Basis von polymerisierbaren Dimersäure-Derivaten aus Beispiel 1 und Beispiel 2

Entsprechend der nachfolgend aufgeführten Tabelle 1 wurden Komposite auf der Basis einer Methacrylatmischung mit dem Dimersäure-Dimethacrylat DMDA aus Beispiel 2 (Komposit A, Vergleich) und unter Einbeziehung des ringöffnend polymerisierbaren Dimersäure-Derivats RODA aus Beispiel 1 (Komposit B) mittels eines Kneters (Firma Linden) hergestellt. Von den Materialien wurden entsprechende Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und des Polymerisationsschrumpfs.

**Tabelle 1: Zusammensetzung der Komposite (Angaben in Masse-%)**

| Stoffe | Komposit A Anteile (Gew.-%) | Komposit B Anteile (Gew.-%) |
|---|---|---|
| Bis-GMA¹⁾ | 6, 92 | 6, 92 |
| TEGDMA²⁾ | 4,85 | 4,85 |
| DMDA | 6,21 | - |
| RODA | - | 6,21 |
| Aerosil OX-50³⁾ | 1,00 | 1,00 |
| Glasfüller GM 27884⁴⁾ | 51, 61 | 51, 61 |
| Sphärosil⁵⁾ | 14,39 | 14,39 |
| Ytterbiumtrifluorid⁶⁾ | 14,89 | 14,89 |
| Photoinitiator⁷⁾ | 0,13 | 0,13 |

| | | |
|---|---|---|
| ¹⁾ Bis-GMA (Esschem) ²⁾ Triethylenglycoldimethacrylat (Esschem) ³⁾ Silanisiertes pyrogenes SiO₂ (Degussa) ⁴⁾ Silanisierter Ba-Al-Borosilikatglasfüller (Schott) mit einer mittleren Partikelgröße von 1,5 µm ⁵⁾ SiO₂-ZrO₂-Mischoxid (Tokuyama Soda, mittlere Primärpartikelgröße: 250 nm) ⁶⁾ YbF₃ (Rhone-Poulenc) mit einer mittleren Partikelgröße von 200 nm ⁷⁾ Bis(4-methoxybenzoyl)diethylgermanium | | |

Aus Tabelle 2 ist ersichtlich, dass das Komposit B auf der Basis eines erfindungsgemäßen ringöffnend polymerisierbaren Dimersäure-Derivats im Vergleich zum Komposit A auf der Basis eines Dimersäure-Dimethacrylats bei vergleichbaren mechanischen Eigenschaften einen deutlich reduzierten Polymerisationsschrumpf zeigt.

**Tabelle 2: Kompositeigenschaften**

| Eigenschaft | Komposit A | Komposit B |
|---|---|---|
| Biegefestigkeit (MPa) nach 24 h | 129 | 140 |
| Biegefestigkeit (MPa) nach 24 h WL¹⁾ | 149 | 124 |
| Biege-E-Modul (GPa) nach 24 h | 10,1 | 9,8 |
| Biege-E-Modul (GPa) nach 24 h WL | 9,7 | 8,7 |
| Polymerisationsschrumpf (Vol.-%) | 3,0 | 1,9 |

| | | |
|---|---|---|
| ¹⁾ WL = Wasserlagerung der Prüfkörper | | |

## Patentansprüche

1. Radikalisch polymerisierbares Monomer der Formel (I) 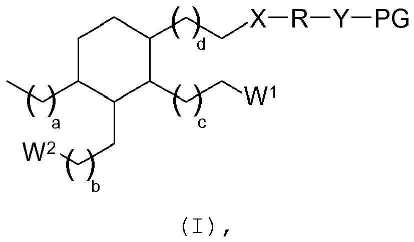 in der
PG jeweils unabhängig für eine cyclische, ringöffnend polymerisierbare Gruppe ausgewählt aus 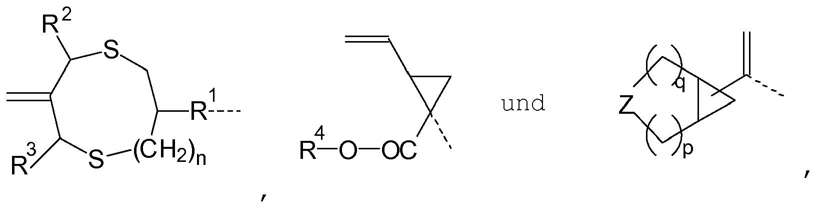 steht,
W¹ und W² jeweils unabhängig für H oder X-R-Y-PG stehen, wobei mindestens eines von W¹ und W² für X-R-Y-PG steht,
X jeweils unabhängig entfällt oder für eine Ether-, Ester-, Amid-, Urethan- oder Harnstoffgruppe steht,
Y jeweils unabhängig entfällt oder für eine Ether-, Ester-, Amid-, Urethan- oder Harnstoffgruppe steht,
Z ausgewählt ist aus O, S, 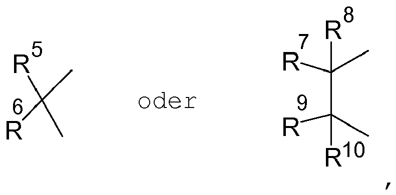
R jeweils unabhängig entfällt oder für einen C₁-C₁₆-Alkylenrest steht, der durch ein oder mehrere O-Atome unterbrochen sein kann, wobei R nur entfallen kann, wenn zugleich X und/oder Y entfällt,
R¹ entfällt oder für einen C₁-C₁₆-Alkylenrest steht, der durch ein oder mehrere O-Atome unterbrochen sein kann,
R² für H oder einen C₁-C₁₀-Alkylrest steht,
R³ für H oder einen C₁-C₁₀-Alkylrest steht,
R⁴ für H oder einen C₁-C₁₀-Alkylrest steht,
R⁵ für H, -CO-OR¹¹, -CO-R¹¹ oder R¹¹ steht,
R⁶ für H, -CO-OR¹¹, -CO-R¹¹ oder R¹¹ steht,
R⁷ bis R¹⁰ jeweils unabhängig für H, -CO-OR¹¹, -CO-R¹¹, ₑi-nen C₁-C₁₅-Alkylrest, der durch ein oder mehrere O-Atome unterbrochen sein kann, einen cycloaliphatischen C₄-C₁₂-Rest, einen bicyclischen C₅-C₁₂-Rest, einen C₆-C₁₄-Arylrest oder einen C₇-C₂₀-Arylalkyl-Rest stehen,
R¹¹ für einen C₁-C₁₅-Alkylrest, der durch ein oder mehrere O-Atome unterbrochen sein kann, einen cycloaliphatischen C₄-C₁₂-Rest, einen bicyclischen C₅-C₁₂-Rest, einen C₆-C₁₄-Arylrest oder einen C₇-C₂₀-Arylalkyl-Rest steht,
a, b, c und d unabhängig voneinander die Werte 3 bis 10 annehmen können,
n 0 oder 1 ist,
p 0, 1, 2 oder 3 ist und
q 0, 1, 2 oder 3 ist.

2. Monomer nach Anspruch 1, bei dem
W¹ für X-R-Y-PG steht,
W² für H steht,
X jeweils unabhängig entfällt oder für eine Ether-, Ester-, Amid-, oder Urethangruppe steht,
Y jeweils unabhängig entfällt oder für eine Ether-, Ester-, Amid-, oder Urethangruppe steht,
R jeweils unabhängig entfällt oder für einen C₁-C₁₂-Alkylenrest steht, der durch ein oder mehrere O-Atome unterbrochen sein kann, wobei R nur entfallen kann, wenn zugleich X und/oder Y entfällt, und
a, b, c und d unabhängig voneinander die Werte 4, 5, 6 und 7 annehmen können.

3. Monomer nach Anspruch 1 oder 2, bei dem
R¹ entfällt oder für einen C₁-C₁₀-Alkylenrest steht, der durch ein oder mehrere O-Atome unterbrochen sein kann,
R² für H oder einen C₁-C₅-Alkylrest steht,
R³ für H oder einen C₁-C₅-Alkylrest steht,
R⁴ für H oder einen C₁-C₅-Alkylrest steht,
R⁵ für H, -CO-OR¹¹, -CO-R¹¹ oder R¹¹ steht,
R⁶ für H, -CO-OR¹¹, -CO-R¹¹ oder R¹¹ steht,
R⁷ bis R¹⁰ jeweils unabhängig für H, -CO-OR¹¹, -CO-R¹¹, einen C₁-C₁₅-Alkylrest, der durch ein oder mehrere O-Atome unterbrochen sein kann, einen cycloaliphatischen C₄-C₆-Rest, einen bicyclischen C₅-C₁₂-Rest, einen Phenyl-Rest oder einen Benzyl-Rest stehen,
R¹¹ für einen C₁-C₁₀-Alkylrest, der durch ein oder mehrere O-Atome unterbrochen sein kann, einen cycloaliphatischen C₄-C₆-Rest, einen Phenyl-Rest oder einen Benzyl-Rest steht,
n 0 oder 1 ist,
p 0, 1 oder 2 ist und
q 0, 1 oder 2 ist.

4. Polymerisierbare Zusammensetzung, die mindestens ein Monomer gemäß einem der Ansprüche 1 bis 3 enthält.

5. Zusammensetzung nach Anspruch 4, die zusätzlich einen Initiator für die radikalische Polymerisation enthält.

6. Zusammensetzung nach Anspruch 4 oder 5, die zusätzlich mindestens ein weiteres radikalisch polymerisierbares Monomer, vorzugsweise mindestens ein weiteres radikalisch polymerisierbares Monomer mit zwei oder mehr radikalisch polymerisierbaren Gruppen enthält.

7. Zusammensetzung nach Anspruch 6, die mindestens ein Monomer enthält, das aus Bisphenol-A-di(meth)acrylat, Bis-GMA, ethoxyliertem Bisphenol-A-di(meth)acrylat, UDMA, Di-, Tri-oder Tetraethylenglycoldi(meth)acrylat, Trimethylolpropan-tri(meth)acrylat, Pentaerythrittetra(meth)acrylat sowie Bu-tandioldi(meth)acrylat, 1,10-Decandioldi(meth)acrylat oder 1,12-Dodecandioldi(meth)acrylat ausgewählt ist.

8. Zusammensetzung nach einem der Ansprüche 6 oder 7, die mindestens ein radikalisch ringöffnend polymerisierbares Monomer enthält, das vorzugsweise aus mono- und multifunktionellen Vinylcyclopropanen, bicyclischen Cyclopropanderivaten oder cyclischen Allylsulfiden ausgewählt ist.

9. Zusammensetzung nach einem der Ansprüche 4 bis 8, die zusätzlich mindestens einen Füllstoff enthält.

10. Zusammensetzung nach einem der Ansprüche 4 bis 9, die weiterhin mindestens ein Additiv enthält, das aus Stabilisatoren, UV-Absorbern, Farbstoffen, Pigmenten, Lösungsmitteln und Gleitmitteln ausgewählt ist.

11. Zusammensetzung nach einem der Ansprüche 4 bis 10, die
(a) 1 bis 90 Gew.-%, bevorzugt 1 bis 80 Gew.-% und besonders bevorzugt 5 bis 70 Gew.-% Monomer der Formel (I),
(b) 0,01 bis 10 Gew.-%, besonders bevorzugt 0,1 bis 3,0 Gew.-% Initiator für die radikalische Polymerisation,
(c) 0 bis 80 Gew.-%, bevorzugt 0 bis 60 Gew.-% und besonders bevorzugt 5 bis 50 Gew.-% weitere radikalisch polymerisierbare Monomere, vorzugsweise 5 bis 80 Gew.-% weiteres ringöffnend polymerisierbares Monomer und 0 bis 50 Gew.-% mehrfach funktionelles (Meth)acrylat,
(d) 0 bis 85 Gew.-% Füllstoff,
(e) ggf. 0,01 bis 50 Gew.-%, bevorzugt 0,05 bis 5 Gew.-% und besonders bevorzugt 0,1 bis 2 Gew.-% Additiv und
(f) 0 bis 95 Gew.-%, bevorzugt 0 bis 70 Gew.-% und besonders bevorzugt 0 bis 50 Gew.-% Lösungsmittel enthält.

12. Verwendung eines radikalisch polymerisierbaren Monomers gemäß einem der Ansprüche 1 bis 3 oder einer polymerisierbaren Zusammensetzung gemäß einem der Ansprüche 4 bis 11 als Dentalmaterial und insbesondere zur Herstellung eines Dentalwerkstoffs.

13. Verwendung nach Anspruch 12, wobei der Dentalwerkstoff ein Komposit, Zement, Adhäsiv oder Beschichtungsmaterial ist.

14. Verfahren zur Herstellung eines Formkörpers, bei dem man eine Zusammensetzung gemäß einem der Ansprüche 4 bis 11 zu einem Körper mit der gewünschten Form formt und anschließend ganz oder teilweise härtet.

## Claims

1. Radically polymerisable monomer of the Formula (I) 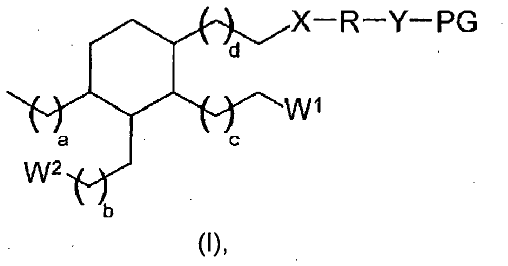 in which
PG in each case independently stands for a cyclic, ring-opening polymerisable group selected from 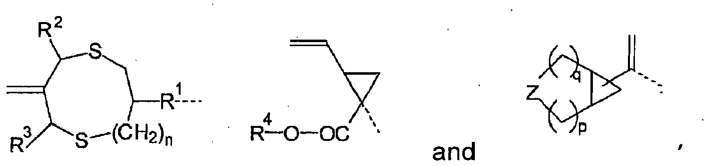 W¹ und W² in each case stand for H or X-R-Y-PG, wherein at least one of W¹ and W² stands for X-R-Y-PG,
X in each case independently is missing or stands for an ether, ester, amide, urethane or urea group,
Y in each case independently is missing or stands for an ether, ester, amide, urethane or urea group,
Z selected from O, S, 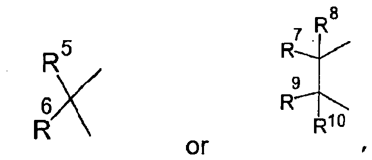
R in each case independently is missing or stands for a C₁-C₁₆ alkylene residue that can be interrupted by one or more O atoms, wherein R can be missing only if X and/or Y are also missing,
R¹ is missing or stands for a C₁-C₁₆ alkylene residue that can be interrupted by one or more O atoms,
R² stands for H or a C₁-C₁₀ alkyl residue,
R³ stands for H or a C₁-C₁₀ alkyl residue,
R⁴ stands for H or a C₁-C₁₀ alkyl residue,
R⁵ stands for H, -CO-OR¹¹, -CO-R¹¹ or R¹¹,
R⁶ stands for H, -CO-OR¹¹, -CO-R¹¹ or R¹¹,
R⁷ to R¹⁰ in each case independently stand for H, -CO-OR¹¹,-CO-
R¹¹ or R¹¹, a C₁-C₁₅ alkyl residue that can be interrupted by one or more O atoms, a cycloaliphatic C₄-C₁₂ residue, a bicyclic C₅-C₁₂ residue, a C₆-C₁₄ aryl residue or a C₇-C₂₀ arylalkyl residue,
R¹¹ stands for a C₁-C₁₅ alkyl residue that can be interrupted by one or more O atoms, a cycloaliphatic C₄-C₁₂ residue, a bicyclic C₅-C₁₂ residue, a C₆-C₁₄ aryl residue or a C₇-C₂₀ arylalkyl residue,
a, b, c and d independently of one another can assume values from 3 to 10,
n is 0 or 1,
p is 0, 1, 2 or 3 and
q is 0, 1, 2 or 3

2. Monomer according to claim 1, in which
W¹ stands for X-R-Y-PG,
W² stands for H,
X in each case independently is missing or stands for an ether, ester, amide, urethane or urea group,
Y in each case independently is missing or stands for an ether, ester, amide, urethane or urea group,
R in each case independently is missing or stands for a C₁-C₁₂ alkylene residue that can be interrupted by one or more O atoms, wherein R can be missing only if X and/or Y are also missing, and
a, b, c and d independently of one another can assume the values 4, 5, 6 and 7.

3. Monomer according to claim 1 or 2, in which
R¹ is missing or stands for a C₁-C₁₀ alkylene residue that can be interrupted by one or more O atoms,
R² stands for H or a C₁-C₅ alkyl residue,
R³ stands for H or a C₁-C₅ alkyl residue,
R⁴ stands for H or a C₁-C₅ alkyl residue,
R⁵ stands for H, -CO-OR¹¹, -CO-R¹¹ or R¹¹,
R⁶ stands for H, -CO-OR¹¹, -CO-R¹¹ or R¹¹,
R⁷ to R¹⁰ in each case independently stand for H, -CO-OR¹¹,-CO-
R¹¹, a C₁-C₁₅ alkyl residue that can be interrupted by one or more O atoms, a cycloaliphatic C₄-C₆ residue, a bicyclic C₅-C₁₂ residue, a phenyl residue or a benzyl residue,
R¹¹ stands for a C₁-C₁₀ alkyl residue that can be interrupted by one or more O atoms, a cycloaliphatic C₄-C₆ residue, a phenyl residue or a benzyl residue,
n is 0 or 1,
p is 0, 1 or 2 and
q is 0, 1 or 2.

4. Polymerisable composition which comprises at least one monomer according to one of claims 1 to 3.

5. Composition according to claim 4 which further comprises an initiator for the radical polymerisation.

6. Composition according to claim 4 or 5 which further comprises at least one additional radically polymerisable monomer, preferably at least one additional radically polymerisable monomer having two or more radically polymerisable groups.

7. Composition according to claim 6 which comprises a monomer that is selected from Bisphenol-A-di(meth)acrylate, Bis-GMA, ethoxylated Bisphenol-A-di(meth)acrylate, UDMA, di-, tri- or tetraethylene glycol di(meth)acrylate, trimethylolpropane tri(meth)acrylate, pentaerythritol tetra(meth)acrylate as well as butanediol di(meth)acrylate, 1,10-decanediol di(meth)acrylate or 1,12-dodecanediol di(meth)acrylate.

8. Composition according to one of claims 6 or 7 which comprises a ring-opening radically polymerisable monomer that is preferably selected from mono and polyfunctional vinylcyclopropanes, bicyclic cyclopropane derivatives or cyclic allyl sulfides.

9. Composition according to one of claims 4 to 8 which additionally comprises at least one filler.

10. Composition according to one of claims 4 to 9, further comprising at least one additive that is selected from stabilisers, UV-absorbers, dyes, pigments, solvents and lubricants.

11. Composition according to one of claims 4 to 10 which comprises
(a) 1 to 90 wt %, preferably 1 to 80 wt % and particularly preferably 5 to 70 wt % monomer of the Formula (I),
(b) 0.01 to 5.0 wt %, particularly preferably 0.1 to 3.0 wt % initiator for the radical polymerisation,
(c) 0 to 80 wt %, preferably 0 to 60 wt % and particularly preferably 5 to 50 wt % of additional radically polymerisable monomers, preferably 5 to 80 wt % of additional ring-opening polymerisable monomer and 0 to 50 wt % of polyfunctional (meth)acrylate,
(d) 0 to 85 wt % filler,
(e) optionally 0.01 to 50 wt %, preferably 0.05 to 5 wt %, and particularly preferably 0.1 to 2 wt % additive,
(f) 0 to 95 wt %, preferably 0 to 70 wt % and particularly preferably 0 to 50 wt % solvent.

12. Use of a radically polymerisable monomer according to one of claims 1 to 3 or of a polymerisable composition according to one of claims 4 to 11 as a dental material and in particular for the preparation of a dental material.

13. Use according to claim 12, wherein the dental material is a composite, cement, adhesive or coating material.

14. Process for manufacturing a moulded article in which process a composition according to one of claims 4 to 11 is moulded into an article with the desired shape, and then partially or completely cured.

## Revendications

1. Monomère polymérisable par voie radicalaire, de formule (I) : 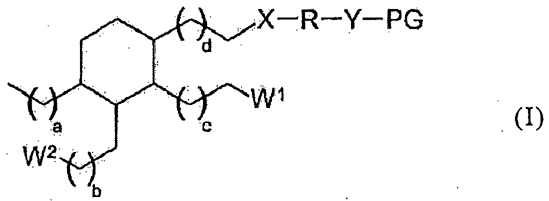 dans laquelle
- PG, indépendamment en chaque occurrence, représente un groupe cyclique polymérisable par ouverture de cycle, choisi parmi ceux de formules 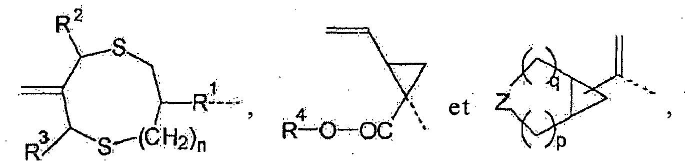
- W¹ et W², indépendamment l'un de l'autre, représentent un atome d'hydrogène ou un groupe symbolisé par X-R-Y-PG, étant entendu qu'au moins l'un de ces symboles W¹ et W² représente un groupe symbolisé par X-R-Y-PG,
- X, indépendamment en chaque occurrence, ne représente rien ou représente un groupe ou chaînon de type éther, ester, amide, uréthane ou urée,
- Y, indépendamment en chaque occurrence, ne représente rien ou représente un groupe ou chaînon de type éther, ester, amide, uréthane ou urée,
- Z représente une entité choisie parmi les atomes d'oxygène ou de soufre et les groupes de formule 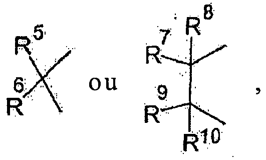
- R, indépendamment en chaque occurrence, ne représente rien ou représente un groupe alcanediyle en C₁-C₁₆, qui peut être inter-rompu par un ou plusieurs atome(s) d'oxygène, étant entendu que R ne peut ne rien représenter que si en même temps X et/ou Y ne représente(nt) rien,
- R¹ ne représente rien ou représente un groupe alcanediyle en C₁-C₁₆, qui peut être interrompu par un ou plusieurs atome(s) d'oxygène,
- R² représente un atome d'hydrogène ou un groupe alkyle en C₁-C₁₀,
- R³ représente un atome d'hydrogène ou un groupe alkyle en C₁-C₁₀,
- R⁴ représente un atome d'hydrogène ou un groupe alkyle en C₁-C₁₀,
- R⁵ représente un atome d'hydrogène ou un groupe symbolisé par -CO-OR¹¹, -CO-R¹¹ ou R¹¹,
- R⁶ représente un atome d'hydrogène ou un groupe symbolisé par -CO-OR¹¹, -CO-R¹¹ ou R¹¹,
- les symboles R⁷ à R¹⁰ représentent chacun, indépendamment les uns des autres, un atome d'hydrogène, un groupe symbolisé par -CO-OR¹¹ ou -CO-R¹¹, un groupe alkyle en C₁-C₁₅ qui peut être interrompu par un ou plusieurs atome(s) d'oxygène, un groupe cyclo-aliphatique en C₄-C₁₂, un groupe bicyclique en C₅-C₁₂, un groupe aryle en C₆-C₁₄ ou un groupe aryl-alkyle en C₇-C₂₀,
- R¹¹ représente un groupe alkyle en C₁-C₁₅ qui peut être interrompu par un ou plusieurs atome(s) d'oxygène, un groupe cycloaliphatique en C₄-C₁₂, un groupe bicyclique en C₅-C₁₂, un groupe aryle en C₆-C₁₄ ou un groupe aryl-alkyle en C₇-C₂₀,
- les indices a, b, c et d peuvent prendre, indépendamment les uns des autres, les valeurs de 3 à 10,
- l'indice n vaut 0 ou 1,
- l'indice p vaut 0, 1, 2 ou 3,
- et l'indice q vaut 0, 1, 2 ou 3.

2. Monomère conforme à la revendication 1, dans lequel
- W¹ représente un groupe symbolisé par X-R-Y-PG,
- W² représente un atome d'hydrogène,
- X, indépendamment en chaque occurrence, ne représente rien ou représente un groupe ou chaînon de type éther, ester, amide ou uréthane,
- Y, indépendamment en chaque occurrence, ne représente rien ou représente un groupe ou chaînon de type éther, ester, amide ou uréthane,
- R, indépendamment en chaque occurrence, ne représente rien ou représente un groupe alcanediyle en C₁-C₁₂, qui peut être inter-rompu par un ou plusieurs atome(s) d'oxygène, étant entendu que R ne peut ne rien représenter que si en même temps X et/ou Y ne représente(nt) rien,
- et les indices a, b, c et d peuvent prendre, indépendamment les uns des autres, les valeurs 4, 5, 6 et 7.

3. Monomère conforme à la revendication 1 ou 2, dans lequel
- R¹ ne représente rien ou représente un groupe alcanediyle en C₁-C₁₀, qui peut être interrompu par un ou plusieurs atome(s) d'oxygène,
- R² représente un atome d'hydrogène ou un groupe alkyle en C₁-C₅,
- R³ représente un atome d'hydrogène ou un groupe alkyle en C₁-C₅,
- R⁴ représente un atome d'hydrogène ou un groupe alkyle en C₁-C₅,
- R⁵ représente un atome d'hydrogène ou un groupe symbolisé par -CO-OR¹¹, -CO-R¹¹ ou R¹¹,
- R⁶ représente un atome d'hydrogène ou un groupe symbolisé par -CO-OR¹¹, -CO-R¹¹ ou R¹¹,
- les symboles R⁷ à R¹⁰ représentent chacun, indépendamment les uns des autres, un atome d'hydrogène, un groupe symbolisé par -CO-OR¹¹ ou -CO-R¹¹, un groupe alkyle en C₁-C₁₅ qui peut être interrompu par un ou plusieurs atome(s) d'oxygène, un groupe cyclo-aliphatique en C₄-C₆, un groupe bicyclique en C₅-C₁₂, un groupe phényle ou un groupe benzyle,
- R¹¹ représente un groupe alkyle en C₁-C₁₀ qui peut être interrompu par un ou plusieurs atome(s) d'oxygène, un groupe cycloaliphatique en C₄-C₆, un groupe phényle ou un groupe benzyle,
- l'indice n vaut 0 ou 1,
- l'indice p vaut 0, 1 ou 2,
- et l'indice q vaut 0, 1 ou 2.

4. Composition polymérisable, qui contient au moins un mono-mère conforme à l'une des revendications 1 à 3.

5. Composition conforme à la revendication 4, qui contient en outre un amorceur pour polymérisation par voie radicalaire.

6. Composition conforme à la revendication 4 ou 5, qui contient en outre au moins un autre monomère polymérisable par voie radicalaire, et de préférence, au moins un autre monomère polymérisable par voie radicalaire et comportant deux groupes, ou plus, aptes à une polymérisation par voie radicalaire.

7. Composition conforme à la revendication 6, qui contient au moins un monomère choisi parmi les suivants : di(méth)acrylate de bisphénol A, bis-GMA, di(méth)acrylate de bisphénol A éthoxylé, UDMA, di(méth)acrylate de diéthylèneglycol, de triéthylèneglycol ou de tétraéthylèneglycol, tri(méth)acrylate de triméthylol-propane, tétra-(méth)acrylate de pentaérythritol, di(méth)acrylate de butanediol, di-(méth)acrylate de décane-1,10-diol, et di(méth)acrylate de dodécane-1,12-diol.

8. Composition conforme à la revendication 6 ou 7, qui contient au moins un monomère polymérisable par ouverture de cycle par voie radicalaire, lequel est choisi, de préférence, parmi les vinyl-cyclo-propanes monofonctionnels ou polyfonctionnels, les dérivés bicycliques du cyclopropane ou les sulfures d'allyle cycliques.

9. Composition conforme à l'une des revendications 4 à 8, qui contient en outre au moins une charge.

10. Composition conforme à l'une des revendications 4 à 9, qui contient en outre au moins un adjuvant, lequel est choisi parmi les stabilisants, absorbeurs UV, colorants, pigments, solvants et agents de glissement.

11. Composition conforme à l'une des revendications 4 à 10, qui contient:
a) de 1 à 90 % en poids, de préférence de 1 à 80 % en poids, et en particulier de 5 à 70 % en poids de monomère de formule (I),
b) de 0,01 à 10 % en poids, et en particulier de 0,1 à 3,0 % en poids d'amorceur pour polymérisation par voie radicalaire,
c) de 0 à 80 % en poids, de préférence de 0 à 60 % en poids, et en particulier de 5 à 50 % en poids d'autres monomères polymérisables par voie radicalaire, de préférence de 5 à 80 % en poids d'un autre monomère polymérisable par ouverture de cycle et de 0 à 50 % en poids d'un (méth)acrylate polyfonctionnel,
d) de 0 à 85 % en poids de charge,
e) en option, de 0,01 à 50 % en poids, de préférence de 0,05 à 5 % en poids, et en particulier de 0,1 à 2 % en poids d'adjuvant(s),
f) et de 0 à 95 % en poids, de préférence de 0 à 70 % en poids, et en particulier de 0 à 50 % en poids de solvant.

12. Utilisation d'un monomère polymérisable par voie radicalaire conforme à l'une des revendications 1 à 3, ou d'une composition polymérisable conforme à l'une des revendications 4 à 11, en tant que matériau dentaire et en particulier pour la préparation d'un matériau de dentisterie.

13. Utilisation conforme à la revendication 12, dans laquelle le matériau de dentisterie est un composite, un ciment, un adhésif ou un matériau de revêtement.

14. Procédé de fabrication d'un corps façonné, dans lequel on façonne un corps de la forme voulue, à partir d'une composition conforme à l'une des revendications 4 à 11, que l'on fait ensuite durcir partiellement ou complètement.
